(19) **Europäisches Patentamt**
**European Patent Office**
**Office européen des brevets**

(11) **EP 3 544 673 B1**

(12) **EUROPEAN PATENT SPECIFICATION**

(45) Date of publication and mention
of the grant of the patent:
**17.11.2021 Bulletin 2021/46**

(51) Int Cl.:
*A61N 1/36* (2006.01)       *A61N 1/05* (2006.01)

(21) Application number: **17873289.7**

(86) International application number:
**PCT/US2017/063225**

(22) Date of filing: **27.11.2017**

(87) International publication number:
**WO 2018/098409 (31.05.2018 Gazette 2018/22)**

(54) **RESPIRATION MONITORING SENSOR FOR A LARYNGEAL PACEMAKER**

ATEMÜBERWACHUNGSSENSOR FÜR EINEN KEHLKOPFSCHRITTMACHER

CAPTEUR DE SURVEILLANCE DE RESPIRATION POUR STIMULATEUR LARYNGÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.11.2016 US 201662426647 P**

(43) Date of publication of application:
**02.10.2019 Bulletin 2019/40**

(73) Proprietor: **Med-El Elektromedizinische Geraete GmbH**
**6020 Innsbruck (AT)**

(72) Inventors:
• **SANTONOCITO, Daniele**
**6020 Innsbruck (AT)**
• **LOMBARDI, Alberto**
**6020 Innsbruck (AT)**
• **SABA, Rami**
**6020 Innsbruck (AT)**
• **MAULE, Francesca**
**6020 Innsbruck (AT)**

• **DIEKOW, Christian**
**6020 Innsbruck (AT)**
• **DENK, Christian**
**6020 Innsbruck (AT)**
• **POSCHL, Christiane**
**6020 Innsbruck (AT)**

(74) Representative: **Lucke, Andreas**
**Boehmert & Boehmert**
**Anwaltspartnerschaft mbB**
**Pettenkoferstrasse 22**
**80336 München (DE)**

(56) References cited:
**US-A- 5 111 814       US-A1- 2002 156 507**
**US-A1- 2012 172 742       US-A1- 2015 283 382**

• **ALESSANDRA ZUCCA ET AL: "Tattoo Conductive Polymer Nanosheets for Skin-Contact Applications", ADVANCED HEALTHCARE MATERIALS, vol. 4, no. 7, 19 February 2015 (2015-02-19), pages 983-990, XP055591408, DE ISSN: 2192-2640, DOI: 10.1002/adhm.201400761**

**Description**

TECHNICAL FIELD

The present invention relates to respiration sensors for laryngeal pacemaker systems.

BACKGROUND ART

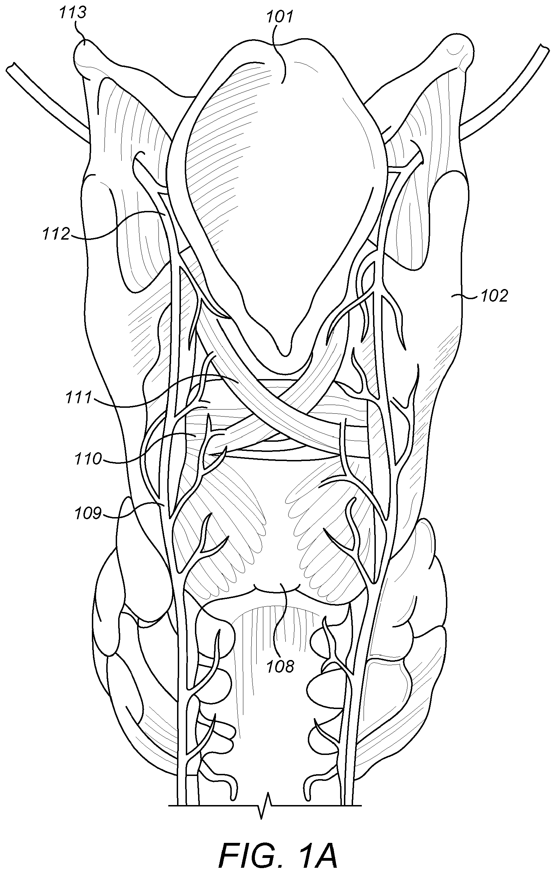

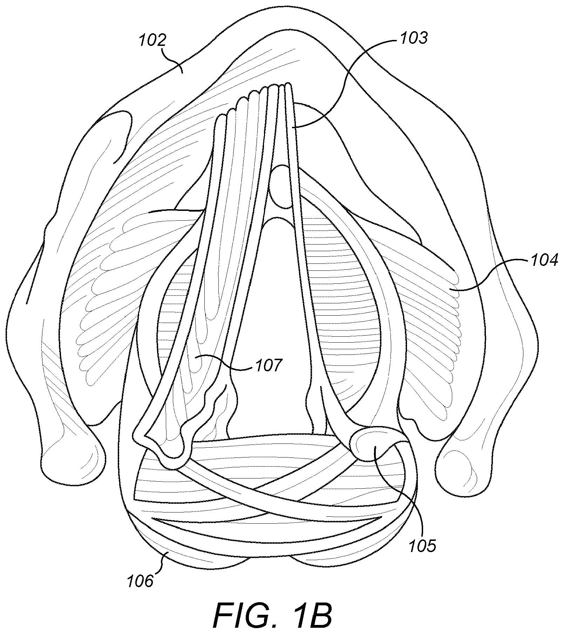

The larynx is located in the neck and is involved in breathing, producing sound (speech), and protecting the trachea from aspiration of food and water. Figure 1A shows a coronal section view and Figure 1B shows a transverse section view of the anatomy of a human larynx including the epiglottis **101**, thyroid cartilage **102**, vocal folds **103**, cricothyroid muscle **104**, arytenoid cartilage **105**, posterior cricoarytenoid muscle (PCAM) **106**, vocalis muscle **107**, cricoid cartilage **108**, recurrent laryngeal nerve (RLN) **109**, transverse arytenoid muscle **110**, oblique arytenoid muscle **111**, superior laryngeal nerve **112**, and hyoid bone **113**.

The nerves and muscles of the larynx abduct (open) the vocal folds **103** during the inspiration phase of breathing to allow air to enter the lungs. And the nerves and muscles of the larynx adduct (close) the vocal folds **103** during the expiration phase of breathing to produce voiced sound. At rest, respiration frequency typically varies from 12 to 25 breaths per minute. So, for example, 20 breaths per minute result in a 3 second breath duration, with 1.5 sec inspiration, and 1.5 sec exhalation phase (assuming a 50/50 ratio). The breathing frequency changes depending on the physical activity.

Unilateral and bilateral injuries or ruptures of the recurrent laryngeal nerve (RLN) **109** initially result in a temporal partial paralysis of the supported muscles in the larynx (and the hypolarynx). A bilateral disruption of the RLN **109** causes a loss of the abductor function of both posterior cricoarytenoid muscles (PCAM) **106** with acute asphyxia and life-threatening conditions. This serious situation usually requires surgical treatment of the bilateral vocal cord paralysis such as cordotomy or arytenoidectomy, which subsequently restrict the voice and puts at risk the physiologic airway protection.

A more recent treatment approach to RLN injuries uses a laryngeal pacemaker that electrically stimulates (paces) the PCAM **106** during inspiration to abduct (open) the vocal folds **103**. During expiration, the vocal folds **103** relax (close) to facilitate voicing. In first generation laryngeal pacemaker systems, the patient can vary the pacing frequency (breaths per minute) according to his physical load (at rest, normal walking, stairs, etc.) by manually switching the stimulation frequency of the pacer device, the assumption being that the human body may adapt to the artificial externally applied respiration frequency - within some locking-range. Thus the patient and the laryngeal pacemaker can be described as free running oscillators at almost the same frequency, but without phase-matching (no phase-locking). Sometimes both systems will be in phase, but other times the systems will be out of phase and thus the benefit for the patient will be reduced.

More recent second generation laryngeal pacemaker systems generate a stimulation trigger signal to synchronize the timing of stimulation of the pacemaker to the respiration cycle of the patient. The stimulation trigger signal defines a specific time point during the respiration cycle to initiate stimulation of the target neural tissue. The time point may specifically be the start or end of the inspiratory or expiratory phase of breathing, a breathing pause, or any other defined time point. To detect the desired time point, several types of respiration sensors have been investigated to generate a respiration sensing signal that varies within each breathing cycle. These include, for example, various microphones, accelerometer sensors, and pressure sensors (positioned in the pleura gap). Electromyogram (EMG) measurements also are under investigation for use in developing a stimulation trigger signal.

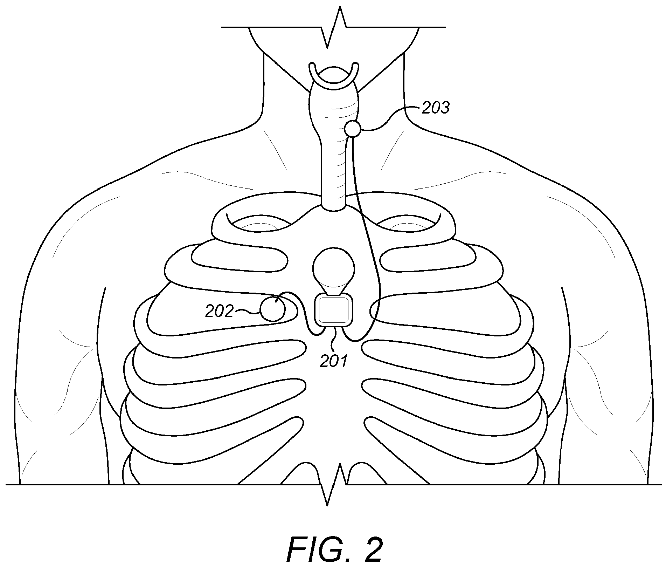

Figure 2 shows one embodiment of such a laryngeal pacemaker system with a processor **201** that receives a respiration signal from a respiration sensor **202** implanted in the parasternal muscle that detects respiration activity in the implanted patient. Optionally, a three-axis acceleration movement sensor also is located within the housing of the processor **201** and generates a movement signal. Based on the respiration signal, the processor **201** generates a respiration pacing signal that is synchronized with the detected respiration activity and delivers the pacing signal via a processor lead to a stimulating electrode **203** implanted in the target respiration neural tissue to promote breathing of the implanted patient.

In conventional laryngeal pacemakers, many different kinds of respiration sensors have been proposed. Many such arrangements require connection to the pacing processor via an insulated conductive wire element embedded in a wire lead. Such a wire lead, though, may be prone to physical damage, requires effort for insertion during surgery, and has to somehow be securely fixed within delicate tissue, e.g. near or around nerves or within muscles.

US 2015/283382 A1 discloses a laryngeal pacing system for a recipient with impaired breathing with an implantable stimulation electrode for delivering the respiration stimulation signals and a triaxial accelerometer, further comprising a pacing processor being implanted in the patient.

## EP 3 544 673 B1

SUMMARY

Embodiments of the present invention are directed to a laryngeal pacing system for a recipient patient with impaired breathing. A laryngeal pacemaker is configured for external placement on skin of a patient at a sternum location and configured to produce respiration stimulation signals. An implantable stimulation electrode is configured for delivering the respiration stimulation signals from the laryngeal pacemaker to adjacent target neural tissue for vocal fold abduction during respiration of the recipient patient. A respiration sensor includes a flexible skin-transferrable printed tattoo electrode having a tetrapolar configuration for impedance pneumography measurement to produce a sensed respiration signal for the laryngeal pacemaker. A triaxial accelerometer is configured to produce a body motion signal for the laryngeal pacemaker that reflects energy expenditure of the recipient patient. The flexible skin-transferrable printed tattoo electrode (PTE) is configured for transfer and release by guided placement from a sensor applicator to a fixed skin location at the angulus sterni of the recipient patient. And the laryngeal pacemaker is configured to interpret the body motion signal and the sensed respiration signal to make a real time determination of respiratory phase and frequency for adaptively adjusting the respiration stimulation signals accordingly.

In a specific embodiment, the laryngeal pacemaker may include an outer surface with sensor contacts configured to directly connect to the PTE for coupling the sensed respiration signal from the respiration sensor to the laryngeal pacemaker. The triaxial accelerometer may be integrated into the respiration sensor. And the printed tattoo electrode may be formed from Tattoo Conductive Polymer Nanosheets for Skin-Contact Applications. The respiration sensor also may include a center support ring configured to mechanically engage the respiration sensor with the laryngeal pacemaker.

The respiration sensor may be configured for transfer and release using a water-based transfer mechanism. For example, there may be a semi-rigid support layer configured to provide mechanical support to the printed tattoo electrode and configured to release a wetting layer of water when mechanically pressed. Such a support layer may include multiple water-holding sub-divisions, or just a single water-holding sub-division. And the respiration sensor may be adapted to cooperate with the sensor applicator to provide positioning feedback information when the respiration sensor is placed at the fixed skin location.

BRIEF DESCRIPTION OF THE DRAWINGS

Figure 1A shows a coronal section view and Figure 1B shows a transverse section view of the anatomy of a human larynx.

Figure 2 shows a typical conventional laryngeal pacemaker arrangement with respect to patient anatomy.

Figure 3 shows a laryngeal pacemaker arrangement with a respiration sensor according to an embodiment of the present invention.

Figures 4A-4D show various structural details associated with a respiration sensor arrangement according to an embodiment of the present invention.

Figure 5 shows schematic details of a respiration sensor according to an embodiment of the present invention.

Figures 6A-6B show various aspects of the principle of IPG measurement according to an embodiment of the present invention.

Figures 7A-7B show various aspects of another specific embodiment of a respiration sensor with a support layer and water pockets according to an embodiment of the present invention.

Figure 8 shows details of a support layer and water pocket according to another embodiment of the present invention.

Figure 9 shows structural details of an alternative bottom surface of a laryngeal pacemaker.

Figure 10 shows various waveforms associated with signal processing of a sensed respiration sensor according to an embodiment of the present invention.

Figures 11A-11B show various structural relationships in a sensor applicator according to an embodiment of the present invention.

Figure 12 shows an exploded view of various structural elements of a sensor applicator according to an embodiment of the present invention.

Figures 13A-13D show various aspects in using a sensor applicator according to an embodiment of the present invention.

DETAILED DESCRIPTION

Most existing respiration monitoring systems can only track the overall respiration rate (RR) over time, and not the true instantaneous respiration phase and frequency in real time. One reason for that is because the measured respiration signal is usually contaminated with a high amount of noise. One of the main sources of this noise in the measured respiratory signal is motion artifacts from physical movement of the sensor electrodes with respect to the tissue being measured. The motion artifacts are in the same frequency range as the respiration signal (0.1 — 1.0 Hz) and as a result cannot be easily filtered out. Consequently, it is difficult to derive a measurement signal that can reliably provide the instantaneous phase and frequency of the respiration.

Various embodiments of the present invention are directed to a laryngeal pacing system for a recipient patient with impaired breathing. RLN stimulation is triggered in phase with the true real-time respiration activity by using a respiration sensor that tracks the instantaneous respiration phase and frequency, communicates it to the electronics of the laryngeal pacemaker which adaptively adjusts the respiration stimulation signals accordingly. A respiration sensor is placed and coupled close to an external portion of the system, i.e. the processor device, which is easy to replace by other identical sensor means. In particular, patients can properly locate and attach the respiration sensor on their own without need of professional assistance. The respiration sensor of the present invention together with proper placement yields a very accurate estimation of the onset of the respiratory phase to which a stimulation signal has to be correlated. Unlike many prior art sensor systems, a particular phase of the respiratory cycle (e.g. the onset) can be reliably detected and not just the overall respiratory rate.

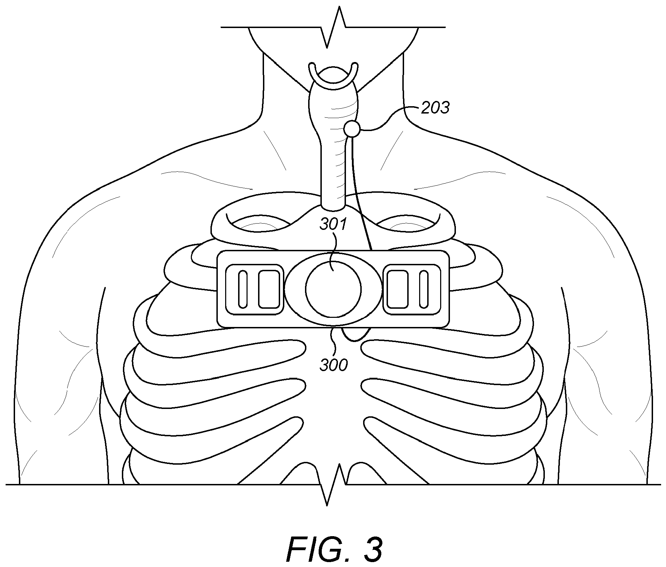

Figure 3 shows an example of a laryngeal pacemaker arrangement with respect to patient anatomy according to an embodiment of the present invention which is configured to provide a reliable respiration signal that reflects real-time instantaneous respiration phase and frequency. A laryngeal pacemaker 301 is configured for external placement on the skin of the patient at as shown in Fig. 3 at the angulus sterni of the patient, and is configured to produce respiration stimulation signals. There is an internal implant portion of the system at that location which includes a holding magnet that cooperates with a corresponding holding magnet in the external laryngeal processor 301 to hold the latter in place. In addition, the angulus sterni location is advantageous because of the ideal flat shape of the bone there, and because there is a low percentage of fat in the underlying skin layer. The thickness of the underlying pectoralis muscle decreases dramatically at the angulus sterni, and the bone there is not part of any joint that might be subject to roll, pitch and yaw. This therefore represents a stable position during periods of high body movement. The width of the manubrium sterni depends on the sex of the subject, but an average width of 5.5 cm can be assumed. That means that changes in the lung impedance due to respiration can be easily measured at the manubrium sterni sides, if at least 5.5 cm distance is given between sensing electrodes located left and right of the angulus sterni. In short, the angulus sterni is an ideal placement for measurement of bio-impedance as discussed further below since motion induced artifacts are considerably reduced. We have identified this position as the best one for measuring reliably an inspiratory signal in the way described below.

As shown in Figure 3, an implantable stimulation electrode 203 as known in the art is configured for delivering the respiration stimulation signals from the laryngeal pacemaker 301 to adjacent target neural tissue (e.g., RLN) for vocal fold abduction during respiration of the patient. The respiration sensor 300 is configured to produce a sensed respiration signal for the laryngeal pacemaker 301. The respiration sensor 300 also is configured for transfer and release by guided placement from a sensor applicator to a fixed skin location at the angulus sterni. The laryngeal pacemaker 301 interprets the body motion signal and the sensed respiration signal to make a real time determination of respiratory phase and frequency for adaptively adjusting the respiration stimulation signals accordingly.

A triaxial accelerometer may be integrated into the respiration sensor 300 or the laryngeal pacemaker 301 to produce a body motion signal for the laryngeal pacemaker 301 that reflects energy expenditure of the patient. The body motion signal also can be used for optimizing battery consumption, and the attending body activity detection can be used to determine steady or low movement situations when the laryngeal pacemaker 301 can switch to a paced stimulation to save power.

The laryngeal pacemaker 301 may be configured to compute Energy Expenditure (EE) as a function of x-, y- and z-axis acceleration signals. The tri-axial acceleration body motion signal reflects both a gravitational component and a body motion component, and so the laryngeal pacemaker 301 may need to initially filter and process the body motion signal to extrapolate only the body motion information. Then a Signal Vector Magnitude (SVM) can be computed and compared to pre-determined thresholds that correspond to different body activities. This activity determination can be used to adjust the parameters of the adaptive filtering and peak detection of the bio-impedance signal explained below.

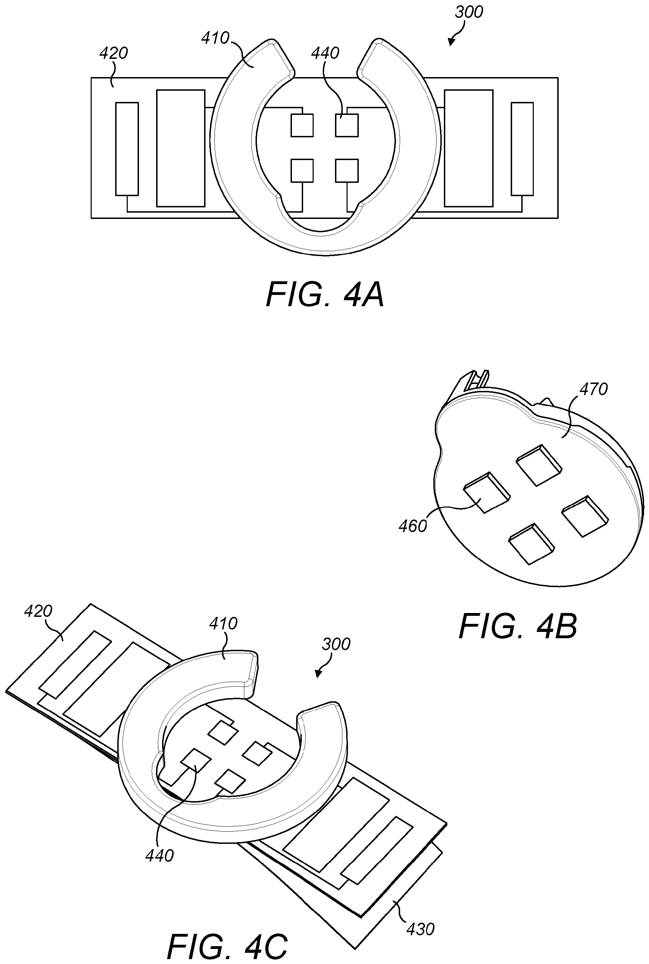

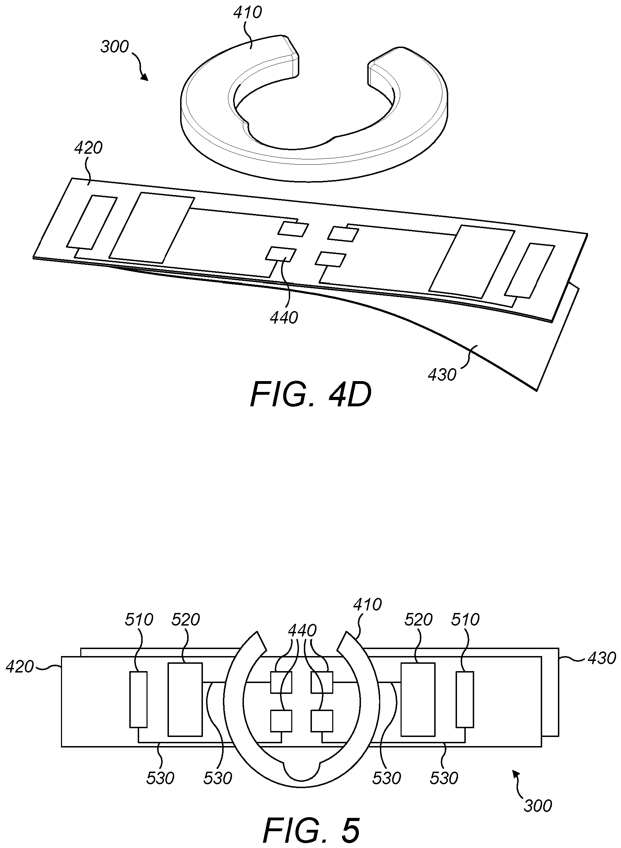

Figures 4A-4D and 5 illustrate structural details of such an arrangement. The respiration sensor **300** includes a center support ring **410** that is configured to mechanically engage the respiration sensor **300** with the laryngeal pacemaker **301**. The specific shape of the support ring **401** can vary to accommodate different specific design features of the housing of the laryngeal pacemaker **301**. Enclosed with the support ring **410** are one or more sensor contacts **440** that are configured to provide a direct electrical connection to corresponding sensor contacts **460** on the bottom surface **470** of the housing of the laryngeal pacemaker **301** when it is enclosed within the support ring **410**.

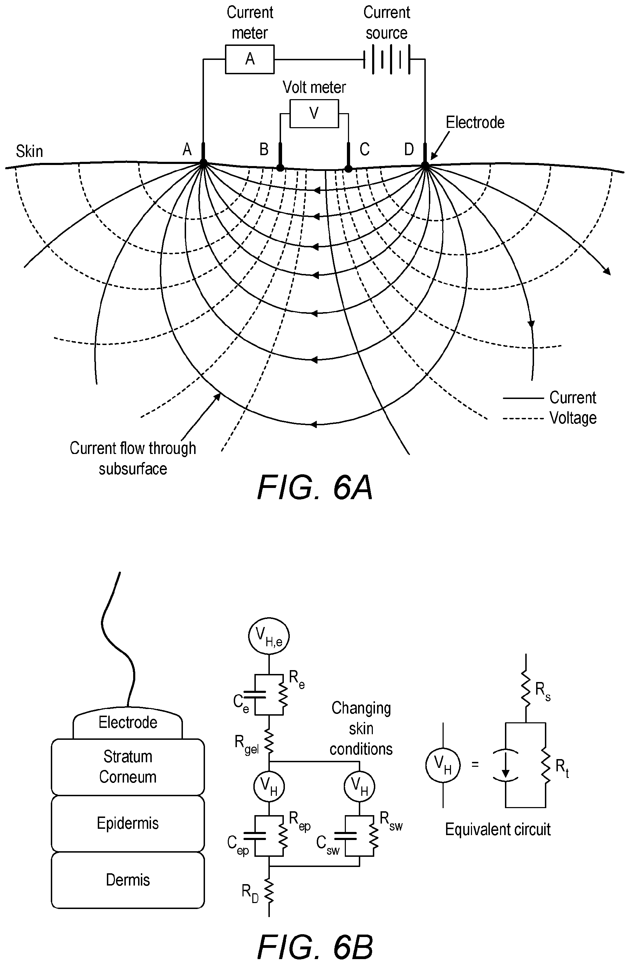

The respiration sensor **300** includes inner sensing contacts **520** and outer excitation contacts **510** that are connected to the sensor contacts **440** by electrically isolated conductive paths **530** made e.g. of gold or other conductive material. The inner sensing contacts **520** and outer excitation contacts **510** are arranged in a tetrapolar configuration for impedance pneumography (IPG) measurement of changes in transthoracic electrical impedance as shown in Figure 6A. Excitation current flows from the outer excitation contacts **510** (A to D), whereas inner sensing contacts **520** (B and C) measure the corresponding difference of voltage. Changes in the sensed voltage reflect impedance changes in the measured tissue region, where the impedance value Z can be obtained from:

$$Z = \int \frac{1}{p} J_{LE} J_{LI} dv$$

where $p$ is conductivity distribution within the volume conductor v, $J_{LE}$ is the lead current density field of voltage measurement, $J_{LI}$ is the current density field raised by current injection.

The electrode-skin interface implicates various considerations with regard to recording biological signals. These include the fact that high skin impedance can result in poor signal detection. In addition, relative movement between the electrode and the skin produces motion artifacts. Motion artifacts result from a change in electrical properties of the skin-electrode interface as shown in Figure 6B. The so-called half-cell potential *VH* (which results from the charge of the metal-electrolyte interface) can be modelled as a current source and parallel resistor Rt. Resistor Rs represents the stratum comeum, which is an outer skin dielectric layer that decreases the quality of the acquired bio-signal. The half-cell potential *VH* arises because the current I flows through the resistive extracellular medium Rt. Motion artifacts therefore appear as a potential change due to the current I flowing through the changing resistance Rt which can increase or decrease depending on the nature of the force applied. The relative movement of the electrode with respect to skin can further change the voltage *VH*. Filtering out and/or reducing motion artifacts is very important.

Wet gel electrodes are commonly used to improve or stabilize the sensing contact and reduce skin impedance by increasing the conductive of the stratum corneum layer. Any mechanical disturbances caused by relative motion between the electrode and the skin are damped by the intervening gel layer, and their effect on the signal is limited. They can be schematized as almost resistive impedance, whose value is in the range of few decades of Ohms. The equivalent impedance *Zequi* derived from Figure 6B therefore can be expressed as:

$$Z_{equi} = R_e \parallel C_e + R_{gel} + R_s + R_t + R_{epi} \parallel C_{epi} + R_d$$

where $R_e$, $C_e$ and $R_{gel}$ all depend on the specific type of electrode and its coupling with the skin. They can change during body movement and still create motion artifacts, although the changed value is reduced as long as the wetting gel does not dry off. When the gel does dry off, the value of $R_{gel}$ increases and the coupling with the skin dramatically decreases. Therefore, long term measurements (i.e. experiments over more consecutive days) are not possible when using standard gel electrodes.

That issue can be addressed if the respiration sensor **300** is made from ultrathin and ultra-conformable flexible nanosheets composed of conducting polymer complex poly (PEDOT:PSS) that form a printed tattoo electrode **420** which provide ultra-conformability on a complex surface such as skin. Using such a material, the respiration sensor **300** specifically may form Tattoo Conductive Polymer Nanosheets for Skin-Contact Applications temporary printed tattoos that are transferred and released to the skin location, thereby overcoming the issue with lack of conformability and poor adhesion that usually occurs with standard dry electrodes.

On the underside of the respiration sensor **300** is an electrode liner **430** that is removed when applying the respiration sensor **300** to the skin. When the electrode liner **430** is removed, the respiration sensor **300** is released to the skin by gently and uniformly rubbing a wet finger (or any other equivalent means) over the top surface of the respiration sensor **300**. The consequent release of the respiration sensor **300** to the skin will occur in a few seconds.

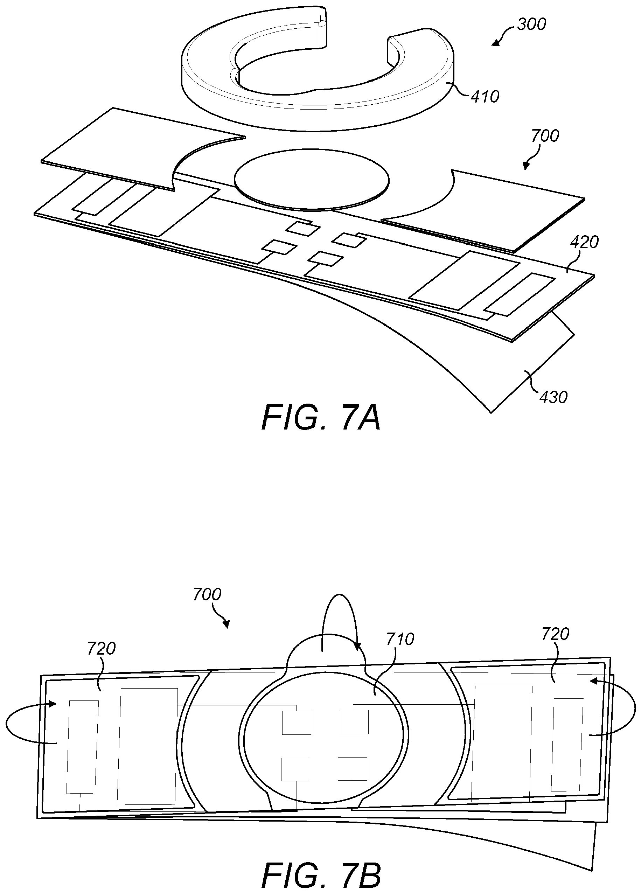

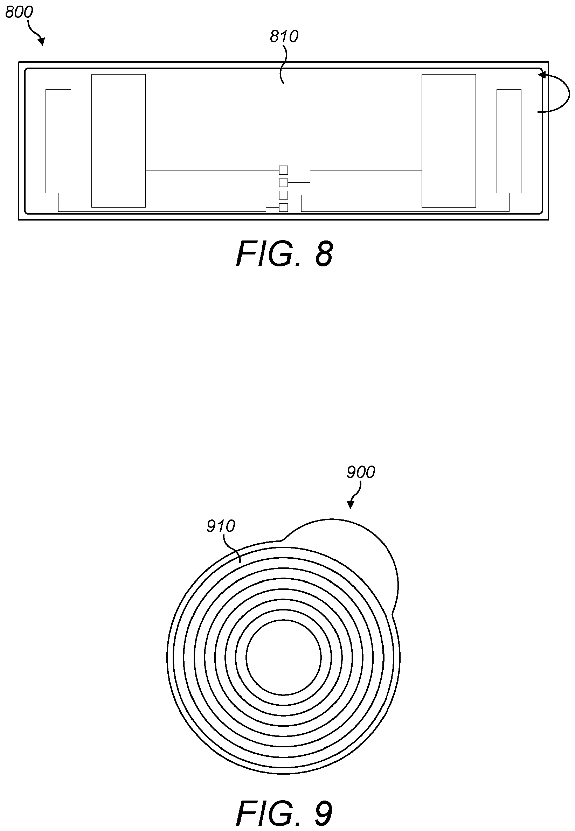

In the embodiment of a respiration sensor **300** shown in Figures 7A-7B, there is an additional semi-rigid support layer **700** that lies between the support ring **410** and the printed tattoo electrode **420** that is configured to provide mechanical support to the printed tattoo electrode **420**. The support layer **700** includes an inner water pocket **710** and two outer water pockets **720** that form an integrated water release system to release a wetting layer of water when mechanically pressed to further promote transfer of the printed tattoo electrode **420** to the skin. The water pockets **710** and **720** are configured to break and release water when finger pressure is applied. After the water has been uniformly released to the underlying printed tattoo electrode **420**, the support layer **700** can be torn off as shown by the arrows. While the support layer shown in Figures 7A-7B includes a plurality of water pockets, an embodiment as shown in Figure 8 could have a support layer **800** with a single water pocket **810**.

Figure 9 shows the bottom surface **900** of a laryngeal pacemaker with sensor contacts **910** in the form of multiple concentric rings. This arrangement would allow the laryngeal pacemaker to freely rotate about its axis while still be able to acquire the sensed respiration signal.

The laryngeal pacemaker **301** performs adaptive filtering processing of the bio-impedance sensed respiration signal from the respiration sensor **300** in combination with the body motion activity determination from the accelerometer signal. The sensed respiration signal typically is defined in the range [0.1 Hz — 1 Hz] in dependence on the body activity occurring. Thus the adaptive filtering of the sensed respiration signal is directed achieving the greatest possible Signal to Noise Ratio (SNR) over the different real-life situations that the patient may encounter. Since the motion artifact noise that affects the sensed respiration signal cannot be considered Gaussian, the adaptive filtering may be based on a Least Mean Square Filter (LMS) approach. By definition, the convergence of the error between the actual and desired signal cannot be guaranteed with LMS (as compared to approaches based on a Kalman filter) and only depends on the number of the iterations performed.

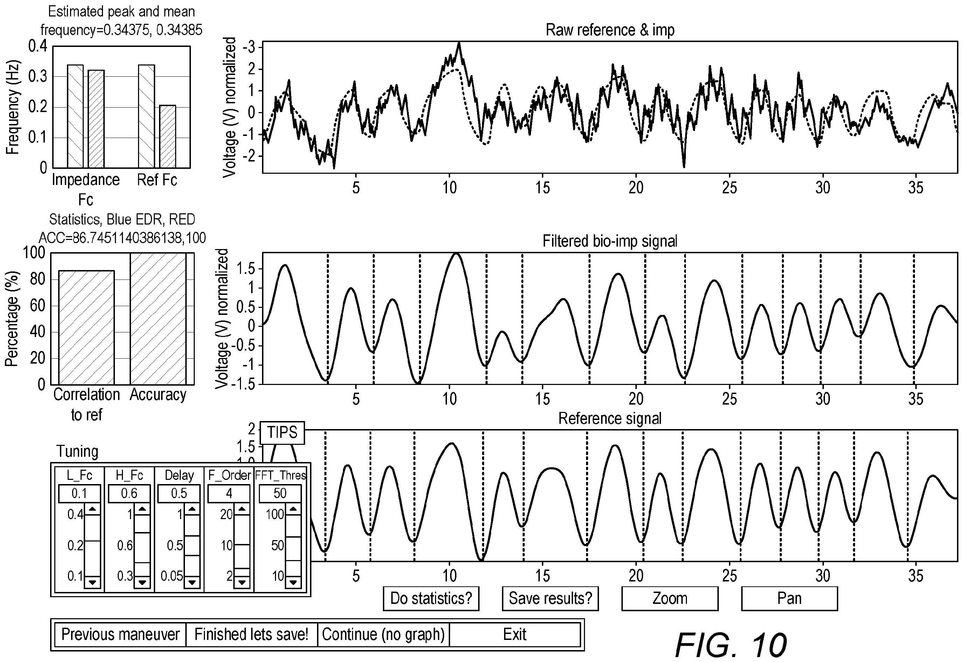

Once the algorithm has been initialized (i.e. learning rate, number of iterations and delay), the sensed respiration signal can be filtered in real time without any further external input. The filtered sensed respiration signal is then used to detect the onset of the respiration as shown in Figure 10. The first row waveform in Fig. 10 shows the raw bio-impedance and reference signals overlapped. The reference signal is acquired by a thermistor that the patient wears during the acquisition together with the sensor and electrodes measuring the bio-impedance. The second and third row waveforms show the filtered bio-impedance and reference signal respectively. The respiration onsets of the signals are shown by the vertical lines on the waveforms. Accuracy detection is computed together with signal correlation in the histogram plots on the left side of Fig. 10 (100% and 94% respectively for this particular experiment). Respiration onset detection is considered successful when the onset in the bio-impedance signal is detected within a maximum delay of 500 msec from the true onset shown in the reference signal. A Bayesian peak validation algorithm can later be used to identify the true peaks based on a Kalman filter.

Embodiments of a laryngeal pacemaker and respiration sensor as described above allow for real time stimulation signals to the target neural tissue that can be triggered by the real time respiratory signal rather than on the base of pre-determined pacing. In addition, the described respiration sensor is not invasive and can be embedded/integrated into the housing of the laryngeal pacemaker without increasing its external dimensions. Moreover, long term monitoring can be provided since the set up the printed tattoo electrode overcomes the issues of wet gel electrodes and can provide ultra-conformability and adhesion on the skin for up to three days. The extremely reduced thickness of the tattoo electrode and the absence of adhesive material also provides for increased comfort and wearability of the respiration sensor. And the tetrapolar electrode configuration together with the placement on the chest at the angulus sterni provides increased robustness to motion artifacts.

The respiration sensor technology described above that is based on Printed Tattoo Electrodes (PTE) can provide ultra-conformability on a complex surface like skin. Their transfer and release as temporary tattoos overcomes the issue with lack of conformability and poor adhesion which usually occurs with standard dry electrodes. And the general field of flexible and skin-transferable sensors is itself receiving ever greater interest due to the potential of developing highly integrated sensors for monitoring vital signs that are portable and not overly invasive.

However, one limiting factor preventing more widespread use of this technology lies in the complexity of the electrode release process. It is difficult to provide a uniform pre-determined release of water onto the tattoo surface, and that together with the need for extra-care in handling such a soft and floppy material often requires an expertly trained subject to facilitate the transfer and release process. The sensor has to be pressed onto the skin strongly enough to provide adequate contact, but not too strongly so as to prevent breakage. In addition, a replacement sensor needs to be applied in the same way as the previously removed one, in the preferred horizontal position in order to properly attach to the skin on both sides of the angulus sterni.

Thus, embodiments of the present invention include a sensor applicator configured to provide reliable, easy and effective placement and release of the sensor by means of a user-friendly design that avoids mis-application. The transfer and release of the PTE sensor can be performed by the patients themselves without needing additional assistance of a trained expert. The sensor applicator guides the patient to correctly place the PTE sensor in the desired correspondence with the angulus sterni in an exactly horizontal positioning, and the sensor applicator can provide feedback to the patient on whether or not the transfer and release process occurred correctly. Embodiments of such a sensor applicator can be used for the placement of any flexible and skin-transferable sensor.

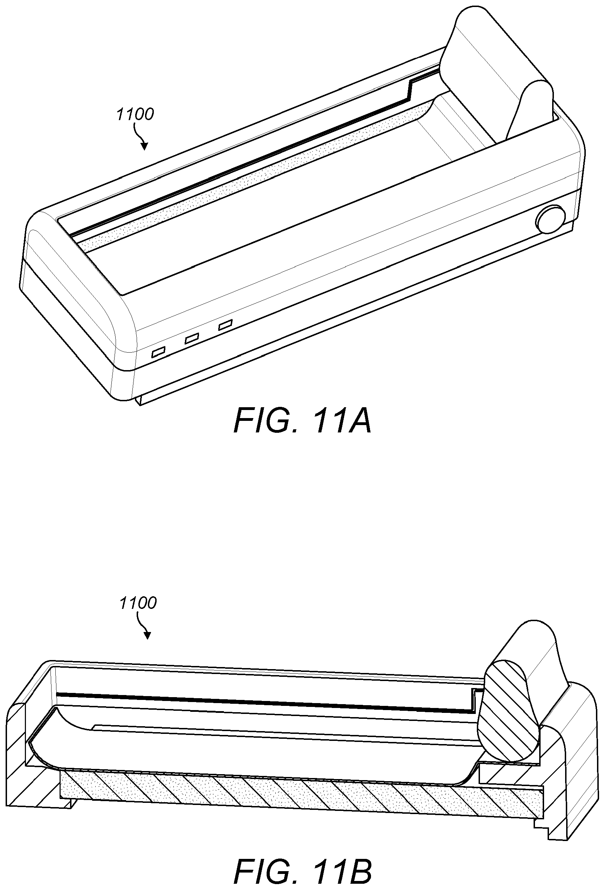

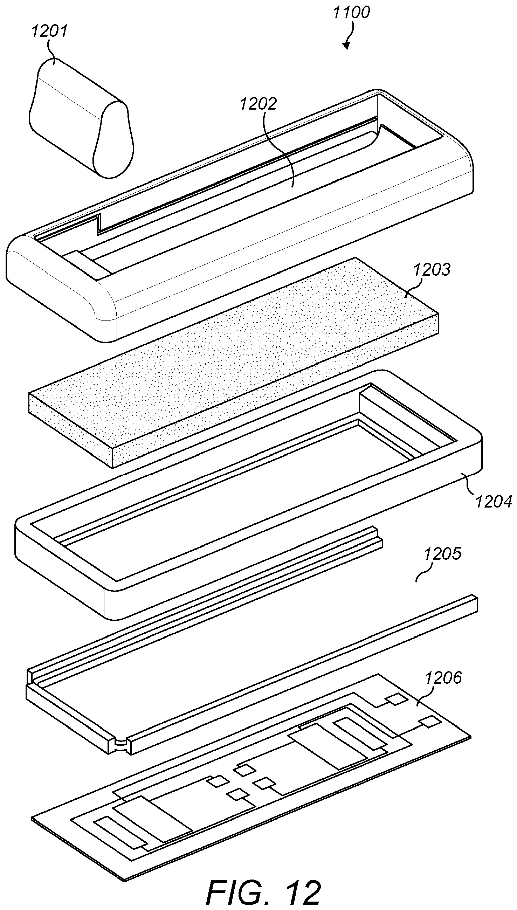

Figures 11A-11B show different views of a sensor applicator **1100** according to an embodiment of the present invention. Specific sensor applicator arrangements may make use of active electronics, passive electronics only, or no electronics at all. Figure 12 shows an exploded view of different structural elements the sensor applicator **1100**. A pressure roller **1201** fits and slides within an upper housing **1202**. Lower housing frame **1204** and bottom surface **1205** fit together to form a holding receptacle configured to contain a fluid storage sponge **1203**. For example, fluid storage sponge **1203** may be made of sponge material or any other equivalent material that can absorb and hold a small amount of release fluid such as water. The PTE sensor **1206** is inserted into the bottom of the sensor applicator **1100**.

Pressure roller **1201** fits within two sliding tracks located within the upper housing **1202** to slide freely along the longitudinal axis of the upper housing **1202**. The bottom side of the upper housing **1202** is a flexible pressure surface on top of which the pressure roller **1201** can be pushed along its displacement trajectory. In specific embodiments, the pressure surface of the upper housing **1202** may be a simple water resistant foil, or in more advanced embodiments, it may be made of a piezo-chromic material that exhibits a reversible color change under pressure effect. Reversible piezo-chromic materials with memory-effect change color at some initial activation pressure PI, and then recover the original color when the applied pressure drops below second recovery pressure P2. The difference between these two pressures P1 and P2 defines the memory effect and allows specification of the history of the material (i.e. if the material has exceeded a threshold of pressure). Once the activation pressure P1 needed for an optimal release of the PTE sensor **1206** onto the skin has been applied using the pressure roller **1201,** the color change of the piezo-chromatic material of the bottom surface can provide user feedback on whether the applied pressure was sufficient and/or uniform enough to guarantee a successful release (or not).

Besides the color-based pressure surface release feedback arrangement described above, there are other ways to provide user feedback about the release process. For example, the upper housing **1202** and/or the lower housing frame **1204** may incorporate signal acquisition sensors configured to receive the sensed ECG signal from the released PTE sensor **1206** when it is applied to the patient's skin. Simple electronics within the sensor applicator **1100** then can perform a signal level thresholding and determine whether or not an acceptable signal has been detected. If acceptable, a feedback LED on the body of the sensor applicator **1100** turns green to confirm the successful release of the PTE sensor **1206**.

Lower housing frame **1204** and bottom surface **1205** fit may be structurally separate pieces configured to fit together, or they may be integrated together into a single structural element. In either case, the bottom surface **1205** is configured to securely hold the PTE sensor **1206** while it is within the sensor applicator **1100**. For example, the bottom surface **1205** may incorporate one or more locking clips arranged to move cooperatively or independently, for example, along a perpendicular axis of the bottom surface **1205**. Such locking clips are configured to provide appropriate support to avoid the PTE sensor **1206** inadvertently slipping away from the bottom surface **1205**. For example, the locking clips can have a saw tooth profile that enhances the security of the PTE sensor **1206** to the bottom surface **1205**.

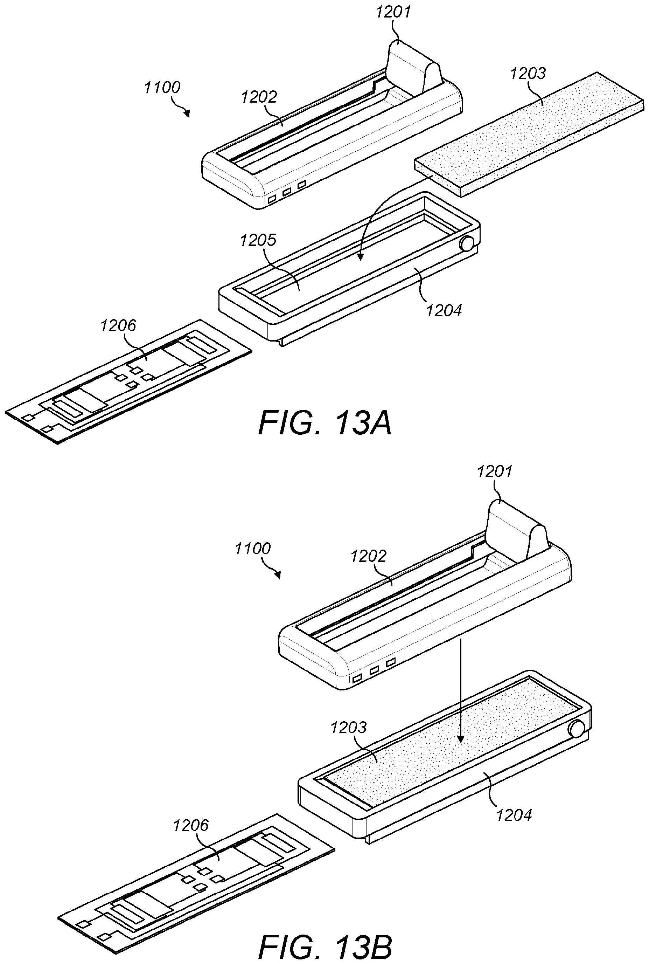

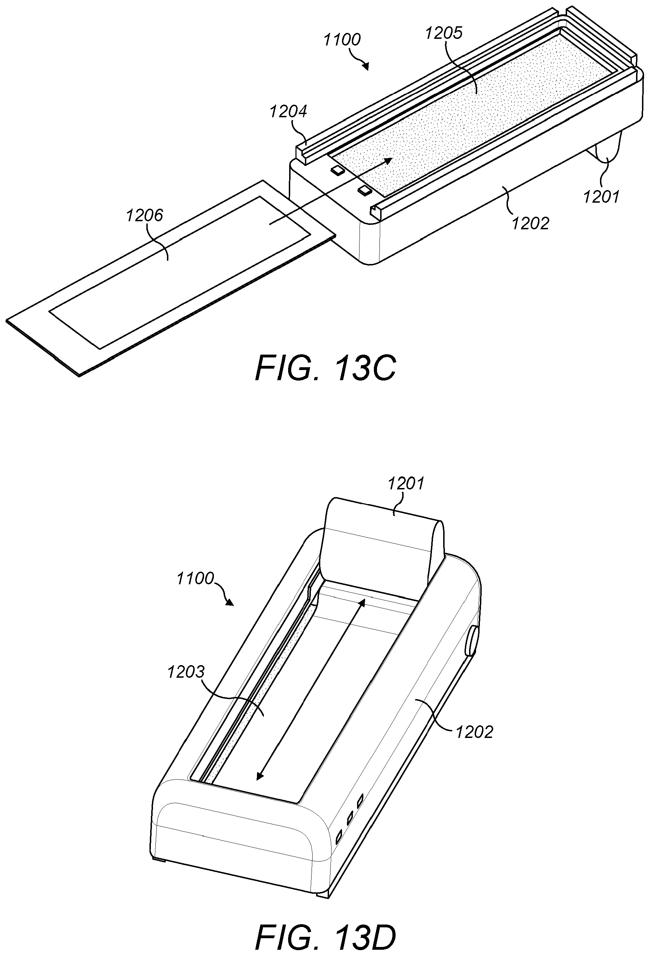

Figures 13A-13D show various aspects in using a sensor applicator according to an embodiment of the present invention. Initially, as shown in Fig. 13A, the sensor applicator **1100** is disassembled so that the fluid storage sponge **1203** needs to be inserted into the lower housing frame **1204** as shown by the thick arrow, and then, as shown by Fig. 13B, the upper housing **1202** lower housing frame **1204** are coupled together. The sensor applicator **1100** can then be inverted and the PTE sensor **1206** can be inserted into the lower housing frame **1204** against the bottom surface **1205** as shown in Fig. 13C. At this point, the electrode liner **430** is removed. Finally, the assembled sensor applicator **1100**, as shown in Fig. 13D, is ready to be placed on the chest of the patient.

Correct placement of the assembled sensor applicator **1100** at the angulus sterni can be confirmed by one or more LEDs in the side of the sensor applicator **1100**. For example, a magnetic sensor within the sensor applicator **1100** can be configured to detect proximity of the device to the implanted magnet at the angulus sterni location, and first positioning LED would then turn green. Moreover, the PTE sensor needs to be placed as closely as possible to horizontal. For that purpose, the sensor applicator **1100** may also contain a sensing gyroscope configured to detect when the device is positioned horizontally within some pre-determined angle of tolerance, and a second positioning LED would then turn green confirming the horizontal position.

At this point, the patient is holding the loaded sensor applicator **1100** on the chest with one hand, and the other hand can then move the pressure roller **1201** within the sensor applicator **1100** to activate the water-based release mechanism. In some specific embodiments, the release mechanism may be further promoted if the patient also pushes down on the pressure roller **1201**. Activation of the pressure roller **1201** squeezes the fluid storage sponge **1203** within the sensor applicator **1100**, causing a uniform water release onto the PTE sensor **1206** that transfers it to the skin. Depending on the specific release feedback mechanism (as discussed above) the patient gets release feedback on whether or not the release movement needs to be repeated. For example, the confirming color change of the pressure surface of the upper housing **1202** or a third release LED turning green confirms a correct conclusion of the sensor application process.

As explained above, specific embodiments of the sensor applicator **1100** may incorporate active electronic elements such as a magnetic sensor, a gyroscope, and/or sensor signal threshold detection logic that are configured to support the patient in the correct placement of the device on the chest. However, specific embodiments of the sensor applicator **1100** may in addition or alternative use passive elements for one or more of the same purposes. For example, the proximity of the sensor applicator **1100** to the implanted magnet may be detected by placing another magnet within the applicator at its center. Horizontal placement of the sensor applicator **1100** may be achieved by using a spirit level in the longitudinal side of the device. In addition, the piezo-chromatic effect described above for release feedback already represents a passive approach.

Embodiments of a sensor applicator as described above reflect a user-friendly design that is easy and intuitive to use. This promotes the precision and reliability of the transfer and release of the sensor device by the patients themselves without the need of an additional expert. Moreover, a sensor applicator with a sponge-based water release mechanism may be even better performing than the breakable water pocket solutions described earlier. In addition, a respiration that is applied by a sensor applicator as just described may avoid the need for incorporating a support ring into the sensor device. That would reduce the cost per unit of the sensor device.

Embodiments of the invention may be implemented in part in any conventional computer programming language such as VHDL, SystemC, Verilog, ASM, etc. Alternative embodiments of the invention may be implemented as pre-programmed hardware elements, other related components, or as a combination of hardware and software components.

Embodiments can be implemented in part as a computer program product for use with a computer system. Such implementation may include a series of computer instructions fixed either on a tangible medium, such as a computer readable medium (e.g., a diskette, CD-ROM, ROM, or fixed disk) or transmittable to a computer system, via a modem or other interface device, such as a communications adapter connected to a network over a medium. The medium may be either a tangible medium (e.g., optical or analog communications lines) or a medium implemented with wireless techniques (e.g., microwave, infrared or other transmission techniques). The series of computer instructions embodies all or part of the functionality previously described herein with respect to the system. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable medium with accompanying printed or electronic documentation (e.g., shrink wrapped software), preloaded with a computer system (e.g., on system ROM or fixed disk), or distributed from a server or electronic bulletin board over the network (e.g., the Internet or World Wide Web). Of course, some embodiments of the invention may be implemented as a combination of both software (e.g., a computer program product) and hardware. Still other embodiments of the invention are implemented as entirely hardware, or entirely software (e.g., a computer program product).

Although various exemplary embodiments of the invention have been disclosed, it should be apparent to those skilled in the art that various changes and modifications can be made which will achieve some of the advantages of the invention without departing from the scope of the invention.

**Claims**

**1.** A laryngeal pacing system for a recipient patient with impaired breathing, the system comprising:

a laryngeal pacemaker (301) configured for external placement on skin of a patient at a sternum location and configured to produce respiration stimulation signals;
an implantable stimulation electrode (203) configured for delivering the respiration stimulation signals from the laryngeal pacemaker to adjacent target neural tissue for vocal fold abduction during respiration of the recipient patient;
a triaxial accelerometer configured to produce a body motion signal for the laryngeal pacemaker reflecting energy expenditure of the recipient patient;
a respiration sensor (300) comprising a flexible skin-transferrable printed tattoo electrode having a tetrapolar configuration for impedance pneumography measurement to produce a sensed respiration signal for the laryngeal pacemaker;
wherein the respiration sensor is configured for transfer and release by guided placement from a sensor applicator to a fixed skin location at the angulus sterni of the recipient patient; and
wherein the laryngeal pacemaker is configured to interpret the body motion signal and the sensed respiration signal to make a real time determination of respiratory phase and frequency for adaptively adjusting the respiration stimulation signals accordingly.

**2.** The laryngeal pacing system according to claim 1, wherein the laryngeal pacemaker includes an outer surface having a plurality of sensor contacts configured to directly connect to the respiration sensor for coupling the sensed respiration signal from the respiration sensor to the laryngeal pacemaker.

3. The laryngeal pacing system according to claim 1, wherein the triaxial accelerometer is integrated into the laryngeal pacemaker.

4. The laryngeal pacing system according to claim 1, wherein the printed tattoo electrode comprises tattoo conductive polymer nanosheets.

5. The laryngeal pacing system according to claim 1, wherein the respiration sensor further comprises a center support ring configured to mechanically engage the respiration sensor with the laryngeal pacemaker.

6. The laryngeal pacing system according to claim 1, wherein the respiration sensor is configured for transfer and release using a water-based transfer mechanism.

7. The laryngeal pacing system according to claim 6, wherein the respiration sensor further comprises a semi-rigid support layer configured to provide mechanical support to the printed tattoo electrode and configured to release a wetting layer of water when mechanically pressed.

8. The laryngeal pacing system according to claim 7, wherein the support layer includes a plurality of water-holding sub-divisions.

9. The laryngeal pacing system according to claim 7, wherein the support layer includes a single water-holding sub-division.

10. The laryngeal pacing system according to claim 1, wherein the respiration sensor is adapted to cooperate with the sensor applicator to provide positioning feedback information when the respiration sensor is placed at the fixed skin location.

**Patentansprüche**

1. Kehlkopfschrittmachersystem für einen Patienten mit beeinträchtigter Atmung, wobei das System folgendes aufweist:

   einen Kehlkopfschrittmacher (301), der zur externen Platzierung auf der Haut eines Patienten an einer Stelle des Brustbeins eingerichtet ist und dazu eingerichtet ist, Atmungsstimulationssignale zu erzeugen;
   eine implantierbare Stimulationselektrode (203), die dazu eingerichtet ist, die Atmungsstimulationssignale von dem Kehlkopfschrittmacher an benachbartes neurales Zielgewebe zur Stimmlippenabduktion während der Atmung des Empfängerpatienten zu liefern;
   einen triaxialen Beschleunigungsmesser, der dazu eingerichtet ist, ein Körperbewegungssignal für den Kehlkopfschrittmacher zu erzeugen, das den Energieverbrauch des Empfängerpatienten widerspiegelt;
   einen Atmungssensor (300), der eine flexible, auf die Haut übertragbare, gedruckte Tattoo-Elektrode mit einer tetrapolaren Konfiguration zur Impedanzpneumographie-Messung aufweist, um ein erfasstes Atmungssignal für den Kehlkopfschrittmacher zu erzeugen;
   wobei der Atmungssensor dazu eingerichtet ist, durch geführte Platzierung von einem Sensorapplikator an eine feste Hautstelle am Angulus sterni des Empfängerpatienten übertragen und freigegeben zu werden; und
   wobei der Kehlkopfschrittmacher dazu eingerichtet ist, das Körperbewegungssignal und das erfasste Atmungssignal zu interpretieren, um eine Echtzeitbestimmung der Atmungsphase und -frequenz vorzunehmen, um die Atmungsstimulationssignale entsprechend adaptiv anzupassen.

2. Kehlkopfschrittmachersystem nach Anspruch 1, wobei der Kehlkopfschrittmacher eine Außenfläche mit einer Vielzahl von Sensorkontakten umfasst, die dazu eingerichtet sind, direkt mit dem Atmungssensor verbunden zu werden, um das erfasste Atmungssignal vom Atmungssensor mit dem Kehlkopfschrittmacher zu koppeln.

3. Kehlkopf-Schrittmachersystem nach Anspruch 1, wobei der triaxiale Beschleunigungsmesser in den Kehlkopf-Schrittmacher integriert ist.

4. Kehlkopfschrittmachersystem nach Anspruch 1, wobei die gedruckte Tattoo-Elektrode leitfähige Polymer-Nanoschichten aufweist.

**5.** Kehlkopfschrittmachersystem nach Anspruch 1, wobei der Atmungssensor ferner einen zentralen Stützring aufweist, der dazu eingerichtet ist, den Atmungssensor mechanisch mit dem Kehlkopfschrittmacher zu verbinden.

**6.** Kehlkopfschrittmachersystem nach Anspruch 1, wobei der Atmungssensor für die Übertragung und Freigabe unter Verwendung eines Übertragungsmechanismus auf Wasserbasis eingerichtet ist.

**7.** Kehlkopfschrittmachersystem nach Anspruch 6, wobei der Atmungssensor ferner eine halbstarre Trägerschicht aufweist, die so eingerichtet ist, dass sie die gedruckte Tattoo-Elektrode mechanisch stützt und so eingerichtet ist, dass sie bei mechanischem Druck eine Benetzungsschicht aus Wasser abgibt.

**8.** Kehlkopfschrittmachersystem nach Anspruch 7, wobei die Trägerschicht eine Vielzahl von wasserhaltenden Unterteilungen umfasst.

**9.** Kehlkopfschrittmachersystem nach Anspruch 7, wobei die Trägerschicht eine einzelne wasserhaltende Unterteilung umfasst.

**10.** Kehlkopf-Schrittmachersystem nach Anspruch 1, wobei der Atmungssensor dazu ausgelegt ist, mit dem Sensorapplikator zusammenzuarbeiten, um eine Positionsrückmeldung zu liefern, wenn der Atmungssensor an der festen Hautstelle platziert ist.

**Revendications**

**1.** Système de stimulation laryngée pour un patient receveur souffrant d'insuffisance respiratoire, le système comprenant :

un stimulateur laryngé (301) conçu pour un placement externe sur la peau d'un patient au niveau d'un emplacement de sternum et conçu pour produire des signaux de stimulation de respiration ;
une électrode de stimulation implantable (203) conçue pour délivrer les signaux de stimulation de respiration du stimulateur laryngé au tissu nerveux cible adjacent pour une abduction de corde vocale au cours de la respiration du patient receveur ;
un accéléromètre triaxial conçu pour produire un signal de mouvement corporel pour le stimulateur laryngé reflétant une dépense énergétique du patient receveur ;
un capteur de respiration (300) comprenant une électrode flexible de tatouage imprimé transférable à la peau ayant une configuration tétrapolaire destinée à la mesure de pneumographie à impédance pour produire un signal de respiration détecté pour le stimulateur laryngé ;
dans lequel le capteur de respiration est conçu pour le transfert et la libération par placement guidé à partir d'un applicateur de capteur à un emplacement de peau fixe au niveau de l'angle sternal du patient receveur ; et
dans lequel le stimulateur laryngé est conçu pour interpréter le signal de mouvement corporel et le signal de respiration détecté pour effectuer une détermination en temps réel de la phase respiratoire et de la fréquence pour ajuster de manière adaptative les signaux de stimulation de respiration en conséquence.

**2.** Système de stimulation laryngée selon la revendication 1, dans lequel le stimulateur laryngé inclut une surface externe ayant une pluralité de contacts de capteur conçus pour se connecter directement au capteur de respiration pour coupler le signal de respiration détecté du capteur de respiration au stimulateur laryngé.

**3.** Système de stimulation laryngée selon la revendication 1, dans lequel l'accéléromètre triaxial est intégré dans le stimulateur laryngé.

**4.** Système de stimulation laryngée selon la revendication 1, dans lequel l'électrode de tatouage imprimé comprend des nanofeuilles de polymère conductrices de tatouage.

**5.** Système de stimulation laryngée selon la revendication 1, dans lequel le capteur de respiration comprend en outre un anneau de support central conçu pour mettre en prise mécaniquement le capteur de respiration avec le stimulateur laryngé.

**6.** Système de stimulation laryngée selon la revendication 1, dans lequel le capteur de respiration est conçu pour le transfert et la libération à l'aide d'un mécanisme de transfert à base d'eau.

**7.** Système de stimulation laryngée selon la revendication 6, dans lequel le capteur de respiration comprend en outre une couche de support semi-rigide conçue pour fournir un support mécanique à l'électrode de tatouage imprimé et conçue pour libérer une couche d'eau de mouillage lorsqu'elle est pressée mécaniquement.

**8.** Système de stimulation laryngée selon la revendication 7, dans lequel la couche de support inclut une pluralité de sous-divisions de rétention d'eau.

**9.** Système de stimulation laryngée selon la revendication 7, dans lequel la couche de support inclut une sous-division de rétention d'eau unique.

**10.** Système de stimulation laryngée selon la revendication 1, dans lequel le capteur de respiration est adapté pour coopérer avec l'applicateur de capteur pour fournir des informations de retour de positionnement lorsque le capteur de respiration est placé au niveau de l'emplacement de peau fixe.

*FIG. 1A*

FIG. 1B

FIG. 2

**FIG. 3**

FIG. 4A

FIG. 4B

FIG. 4C

*300*

*410*

*420*

*440*

*430*

## FIG. 4D

*510*    *520*          *440*    *410*         *520*    *510*

*420*                                                    *430*

*530*    *530*                  *530*    *530*

*300*

## FIG. 5

Current flow through
subsurface

*FIG. 6A*

*FIG. 6B*

**FIG. 7A**

**FIG. 7B**

800

810

FIG. 8

900

910

FIG. 9

FIG. 10

1100

## FIG. 11A

1100

## FIG. 11B

*1100*

*1201*

*1202*

*1203*

*1204*

*1205*

*1206*

## FIG. 12

*FIG. 13A*

*FIG. 13B*

FIG. 13C

FIG. 13D

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015283382 A1